# EUROPEAN PATENT APPLICATION

(11) **EP 4 549 150 A1**
(43) Date of publication of application: **07.05.2025**
(21) Application number: 23020486.9
(22) Date of filing: 30.10.2023
(51) Int. Cl.: B33Y 70/00, C07C 69/75, C08F 220/28, C08F 222/10, C08F 265/06, C08F 267/06

(54) **RESIN COMPOSITION FOR THREE-DIMENSIONAL PRINTING**

(71) Applicant: Cubicure GmbH, 1230 Wien (AT); Align Technology, Inc., San Jose, CA 95134 (US)
(72) Inventor: CONCEICAO, Rafaela, 1170 Vienna (AT); FRANK, Christian, 1230 Vienna (AT); KURY, Markus, 1060 Vienna (AT); GORSCHE, Christian, 7210 Mattersburg (AT); CHOUDHARY, Umesh Upendra, Scotts Valley, CA 95066 (US); SU, Jessica Kalay, San Jose, CA 95116 (US); COLE, Michael Christopher, San Jose, CA 95164 (US)
(74) Representative: SONN Patentanwälte GmbH & Co KG

(57) **Abstract**

Resin composition for three-dimensional printing, the resin composition comprising a) at least one curable oligomer or prepolymer resin component (compound B), and b) a reactive diluent (compound A), the reactive diluent having one or more chemical species being a cycloaliphatic compound according to formula (I):

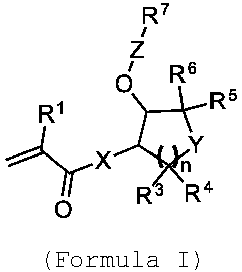

## Description

The present invention relates to a resin composition for three-dimensional printing, and a method of manufacturing an object by 3D-printing.

Reactive diluents are low-molecular weight organic molecules that play a crucial role in fields such as coatings, adhesives and additive manufacturing. They comprise monomers to be mixed in resin compositions to reduce their viscosity and improve processability and upon inducing polymerization, i.e., curing, of the resin composition react covalently with the polymerizing, curing resin composition, becoming part of the final polymer network of the cured matrix.

A specific class of polymerizable monomers suitable as reactive diluents for high temperature processes (>40 °C), such as stereolithography, is employed in the present invention. In addition to low volatility, the specific reactive diluents feature low water uptake, high reactivity and improved (thermo)mechanical properties (e.g., higher T_{g}).

Isobornyl methacrylate (IBMA) and 2-hydroxyethylmethacrylate (HEMA) are two commonly used reactive diluents in industry, which do not comply with all the criteria for an ideal reactive diluent for use in the present invention. HEMA presents low reactivity due to delayed gelation and high water-uptake due to the hydrophilic hydroxy group, jeopardizing the mechanical properties of the polymer network by polymerization of the resin composition. In addition, both molecules are highly volatile, which not only results in a strong, unpleasant smell, but also poses a health risk to the user, especially when they are used in high-temperature 3D printing processes.

Several papers in the literature are directed to addressing the above issues. For example, vanillin, eugenol (ChemSusChem 2012, 5, 1291, ACS Sustainable Chem. Eng. 2017, 5, 8876), carvacol, thymol, menthol (ACS Sustainable Chem. Eng. 2020, 8, 17234), sobrerol, furoic acid (Polymer 2019, 11, 1815), mandelates (US20160347960A1), benzoates (JP2018076435A), and maleates (JP2017110154A) were methacrylated to yield reactive diluents with low volatility at room temperature resulting in polymers with a high T_{g}. However, neither the volatility nor its diluting properties at higher temperatures were addressed.

Methacrylated isopropylidene glycerol (WO 2018146258A1) and tricyclodecane methanol mono (meth)acrylate (US2018194885A1) have also been presented as possible low-volatility reactive diluents, however, in addition to the likely high volatility at higher temperatures, the polymers in question are likely to have low T_{g} (lower than 90°C) .

In WO 2022/272142A1 (meth)acrylated derivatives of syringol, vanillin or guaiacol derivatives, for example, are presented as polymerizable monomers with low vapor pressure at high temperatures (>60°C), however, in some cases these compounds can be solid at around room temperature (melting point (m.p.) >40°C) .

2-hydroxycyclohexyl methacrylate (HCMA) is a low-molecular weight cycloaliphatic methacrylate which has been employed as a strengthening agent in the preparation of hydrogels. It was shown that HCMA is highly reactive and that when added to a hydrogel formulation together with 2-hydroxyethylmethacrylate (HEMA) a material with improved mechanical properties is obtained when compared with hydrogels containing HEMA alone.

Besides its applications in hydrogels, HCMA has found potential in other fields. HCMA has been used, for example, in the preparation of adhesives, coatings or thin films. HCMA has also been employed as a precursor to synthesize urethane methacrylates beforehand, which could afterwards be polymerized to form adhesives. This is disclosed in CN103382382.

In this disclosure a reactive diluent refers to a low molecular-weight, polymerizable, monofunctional monomer that reduces the viscosity of another component, such as a polymer, prepolymer or oligomer. The reactive diluent as used herein can be incorporated into a formulation and when cured, either thermally and/or photopolymerized, becomes part of the obtained polymer. An ideal reactive diluent should, therefore, be liquid at room temperature and low viscous, present a high reactivity with the resin composition, low volatility and high stability at processing temperature. As these molecules become an integral part of the polymer network once the resin composition has cured by polymerization, it is also of utter importance that a reactive diluent has good (thermo)mechanical properties, such as a high glass transition temperature in order to meet the requirements of the cured resin composition.

As used herein a polymer refers to a molecule comprised of a high number of repeating structural units (equal or higher than 10 repeating units and often equal or higher than 50 or even 100 repeating units), bound by a covalent bond and with a high-molecular weight (e.g., equal or higher than 5000 Da, 10000 Da or 20000 Da). In this disclosure, the term "polymer" can be a homopolymer obtained from the polymerization of one species of monomer or a copolymer obtained from the polymerization of 2 or more different species of monomers.

An oligomer as used herein refers to a molecule comprised of a smaller number of covalently bonded repeating structural units (less than a polymer, meaning less than 10 structural units) and a smaller molecular weight than a polymer (less than 5000 Da), resulting from the polymerization of one or more monomers.

As used herein a prepolymer refers to a polymer or an oligomer that can further polymerize due to available reactive groups.

As used herein the term "telechelic oligomer" refers to an oligomer that contains a reactive terminal group on every available chain end and is thus di-, tri- or tetrafunctional or has even a higher number of functional reactive groups and terminal ends thereof. The reactive groups can be the same or different groups.

As used herein, an initiator refers to a chemical compound that can produce radical species, leading to polymerization. This initiator can be either a photoinitiator, meaning a chemical compound that generates the radical upon exposure to UV or visible light, or a thermal initiator, referring to a molecule that forms radicals upon heating above a certain activation temperature.

As used herein, the term "molecular weight" refers to the number average molecular weight (Mₙ) as measured by gel permeation chromatography (GPC), or size exclusion chromatography (SEC) calibrated with polystyrene standards in a suitable solvent, such as tetrahydrofuran.

It is the object of the present invention to provide a resin composition for three-dimensional printing with a reactive diluent of cycloaliphatic compounds, referred to as compounds A, that can be used to reduce the viscosity of the at least one curable oligomer or prepolymer resin component, referred to as compounds B, improving its processability. More concretely, to overcome the problems related with the use of usually applied reactive diluents, the new polymerizable monomers present low volatility, making them suitable for high temperature processes (>40°C) and afford materials with good (thermo)mechanical properties, such as improved T_{g}, (glass transition temperature) and high toughness.

To achieve this object, the resin composition mentioned at the outset, according to the invention, comprises
a) at least one curable oligomer or prepolymer resin component (compound B), and
b) a reactive diluent (compound A), the reactive diluent having one or more chemical species being a cycloaliphatic compound according to formula (I): 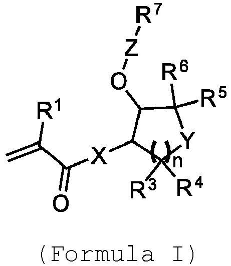 wherein:
   n is an integer between 1 and 5;
   R¹ is a hydrogen or a methyl group;
   X is an -O- or -NR₂-;
   R² is an H or an alkyl group;
   R³, R⁴, R⁵ and R⁶ are independently H, a linear or branched C1-C20 aliphatic group or a C5-C8 cycloaliphatic group; or R³ and R⁴ together and/or R⁵ and R⁶ together represent a carbonyl group;
   Y is carbon, oxygen, sulfur, sulfone, or absent;
   Z is a carbonyl group, or forms a carbonate (-CO-O-) or carbamate group (-CO-NR⁸), or is absent;
   R⁷, R⁸ are independently H, a linear or branched C1-C20 aliphatic group, C5-C8 cycloaliphatic group, a heterocycle such as a tetrahydrothiophene, or a substituted or unsubstituted aromatic group, wherein the substituents of the aromatic group can be H, a linear or branched C1-C10 aliphatic group, methoxy or ethoxy groups, or halogens such as F, Cl, Br or I.

The inventive resin composition exhibits improved (thermo)mechanical properties (e.g., T_{g}) and a low tendency for crystallization due to its cycloaliphatic moiety next to the polymerizable (meth)acrylate unit of compound A. These outstanding properties for suitable reactive diluents are based on the reduced rotational freedom for the defined reactive diluent, via the cycloaliphatic compound A of claim 1. A characteristic aspect of the disclosed diluent class according to formula I is its cycloaliphatic nature and resulting substitution pattern - being non-symmetric - which yields compounds A with low tendency for crystallization, thus making these compounds A particularly suitable as reactive diluents. Easy synthetic accessibility of such reactive diluents according to formula I through the efficient ring opening step from an epoxide precursor is another critical advantage for this disclosed diluent class, making it particularly interesting for industrial scale up. In addition, those reactive diluents exhibit higher molar mass, especially when R⁷ is different from H, yielding diluents with low volatility. Thus, such diluents are especially suitable for the use in 3D-printing at elevated temperature (e.g., 40-150 °C).

In formula (I) n is preferably 1 or 2, yielding a reactive diluent with a 5- or 6-membered cyclic ring structure, respectively. These compounds are preferable, as the precursor molecules are more readily available and overall synthesis of such compounds is more easily feasible.

In formula (I) n is preferably 2 or 3 when Y is absent, yielding a reactive diluent with a 5- or 6-membered cycloaliphatic ring structure, respectively. These compounds are preferable, as the precursor molecules are more readily available and overall synthesis of such compounds is more easily feasible.

In a preferred embodiment of the present invention, the polymerizable oligomer, referred to as component B, can be a (meth)acrylate, (meth)acrylamide, vinyl ester, vinyl ether, vinyl amide, vinyl carbonate, vinyl carbamate, styrene, itaconate, fumarate, maleimide and derivatives thereof. These disclosed classes of functional units are susceptible to radical chain growth polymerization, thus tending to undergo copolymerization with the inventive reactive diluents according to formula I typically forming a polymer network.

Preferably, the at least one curable oligomer or prepolymer resin component comprises one or more mono- or multifunctional chemical species. By comprising multifunctional chemical species that undergo radical chain growth polymerization, a crosslinking reaction is taking place yielding the formation of a polymer network.

Preferably, the molecular weight of the at least one curable oligomer or prepolymer resin component is higher than 450 g/mol, preferably higher than 1000 g/mol and more preferably higher than 2000 g/mol. The use of such curable, high molecular weight oligomers, preferably with a T_{g} below ambient conditions, yields tough photopolymer networks via the introduction of long flexible linkers. Herein, when higher amounts of monofunctional reactive diluents are employed in place of crosslinking monomers within the resin formulation to enable processing of such high molecular weight oligomers, toughness is critically improved, yet heat resistance and strength are often compromised. Thus, light-curable oligomeric compounds having higher molecular weights are employed, exhibiting a T_{g} above ambient conditions, which would critically improve printing performance and improve the material performance of the final photopolymer. While (thermo)mechanical performance such as T_{g}, modulus and strength would be enhanced, toughness could be compromised.

In a preferred embodiment, compositions of such oligomers may be comprising two, three or multiple different oligomers as described.

Preferably, the reactive diluent is already used as solvent for the synthesis of the at least one curable oligomer or prepolymer resin component.

Preferably, the amount of reactive diluent ranges from 5 wt% to 80 wt%, preferably from 5 wt% to 60 wt%, more preferably from 5% to 50% and even more preferably from 5 wt% to 40 wt% based on the total weight of the at least one curable oligomer or prepolymer resin component and the reactive diluent. Higher amounts of reactive diluent result in better printability and easier resin handling. In most cases it is beneficial to keep the reactive diluent as low as possible (e.g., from 5 wt% to 40 wt%), as the prominent network forming component is the employed multifunctional oligomer yielding tough polymer networks.

Preferably, the amount of the at least one curable oligomer or prepolymer resin component ranges from 20 wt% to 95 wt%, preferably from 40 wt% to 95 wt% and more preferably from 50 wt% to 95 wt% and even more preferably from 60 wt% to 95 wt% based on the total weight of the at least one curable oligomer or prepolymer resin component and the reactive diluent. While higher amounts of oligomers tend to yield high performance materials with enhanced toughness, printability may suffer.

Preferably, the reactive diluent has a weight loss at a temperature of up to 50°C over 24 hours of lower than 10 %, preferably lower than 5 %, and more preferably lower than 1 %. Disclosed reactive diluents (compounds A) according to formula I exhibit lower volatility and better printability at elevated temperatures compared to state-of-the-art diluents such as HEMA or isobornyl methacrylate. Low volatile reactive diluents are critical for securing uniform properties throughout the printing process and also ensure good worker safety. Moreover, if reactive diluents within the resin composition do not react during the polymerization reaction and happen to be trapped in the final polymer network, disclosed low volatile reactive diluents show reduced tendency for migration due to their molecular size. This tendency toward lower migration is beneficial for applications in the biomedical field.

Preferably, the reactive diluent has a viscosity of 1 to 100 mPa.s, preferably of 1 to 50 mPa.s, and more preferably of 1 to 20 mPa.s at processing temperatures between 30 and 150°C. Typically, it can be expected that the lower the viscosity of the reactive diluent, the better the diluting properties for the final resin. Compounds A according to formula I in particular are expected to exhibit comparatively high viscosities at ambient conditions, yet show low viscosities in the preferred range at elevated temperatures between 30 and 150 °C. This makes the disclosed compounds A particularly useful as reactive diluents for 3D-printing at elevated temperatures in the range of 30 to 150 °C.

Preferably, in formula (I) R⁷ is selected from the following structures, where the dotted lines represent the position of the bond to Z in formula (I):

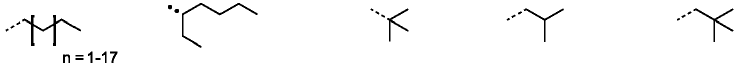

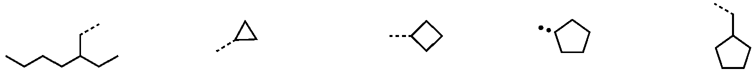

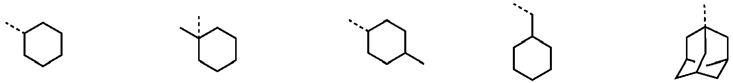

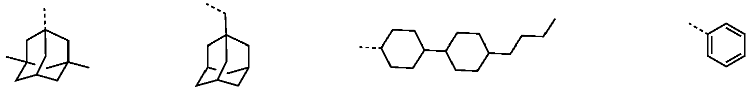

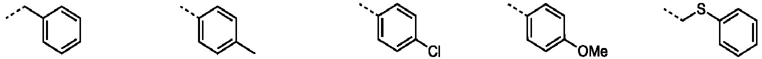

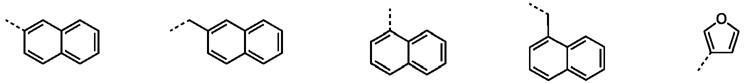

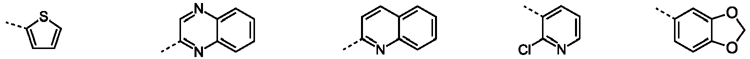

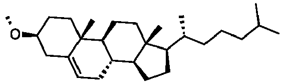

According to a preferred embodiment of the present invention, the reactive diluent according to the general formula (I) is selected from the group comprised of the following compounds:
- 2-hydroxycyclohexyl methacrylate (Ex 1) 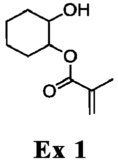
- 2-(cyclohexylcarbonyloxy)cyclohexyl methacrylate (Ex 2) 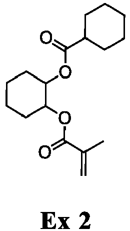
- 2-(isopropenylcarbonyloxy)cyclohexyl 2,2-dimethylpropionate (Ex 3) 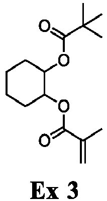
- 2-(benzylcarbonyloxy)cyclohexyl methacrylate (Ex 4) 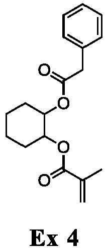
- 2-(isopropenylcarbonyloxy)cyclohexyl 2-ethylhexanoate (Ex 5)

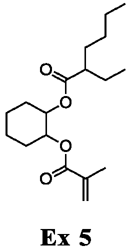

Preferred compounds A exhibit hydrophilic functional groups (e.g., Ex 1 with Z being absent and R⁷ being an H), additional cycloaliphatic (e.g., Ex 2 with Z being a carbonyl group and R⁷ being cyclohexyl), bulky or branched aliphatic (e.g., Ex 3 with Z being a carbonyl group and R⁷ being t-butyl and Ex 5 with Z being a carbonyl group and R⁷ being ethyl-pentyl) or "quasi" aromatic moieties (e.g., meaning that a flexible methylene spacer is introduced, Ex 4 with Z being a carbonyl group and R⁷ being benzyl) as R⁷, which all result in reduced volatility and low tendency for crystallization, making those disclosed compounds ideal candidates for preferred reactive diluents employed in the inventive resin composition for three-dimensional printing.

The present invention achieves its objects for all reactive diluents that fall under the definition claim 1. Therefore, the present invention is not necessarily directed to using 2-hydroxycyclohexyl methacrylate (HCMA, Ex 1) as a reactive diluent.

Preferably, the resin further comprises at least one photoinitiator, preferably suitable for radical polymerization upon light excitation, preferably light excitation within the wavelength spectrum of 150 nm to 1000 nm, more preferably between 200 nm to 550 nm, preferably in amounts of 0.01 wt% to 10 wt%, preferably 0.1 wt% to 7 wt%, more preferably 0.2 wt% to 5 wt%, based on the total weight of the at least one curable oligomer or prepolymer resin component and the reactive diluent in the composition. This means the present photoinitiators in the resin composition can be activated with light and efficiently initiate polymerization of the light-curable components of the formulation during the 3D-printing process. The light-initiated polymerization (photopolymerization) is successful, when the applied light exposure is of a suitable wavelength for activating the photoinitiator and is of sufficient power.

Aside from the light exposure being adequate for the photoinitiator, the overall formulation (optionally including light absorbing monomers or additives) may not interfere with the interaction of the emitted light and the photoinitiator. Light exposure applied herein includes any wavelength and power being able to initiate polymerization. Preferred wavelengths of light are 150 nm to 1000 nm, even more preferred between 200 nm to 550 nm. Any suitable NIR, UV or visible light source may be used, including but not limited to lasers or LED sources or broadband Hg-lamps. The light source may emit a broadband or narrowband light spectrum, or a combination thereof. The light source may emit continuous or pulsed light during single or repeated exposure periods, which may additionally be varied via time or intensity of exposure. A suitable and elevated process temperature will increase the reactivity of the system and thus enhance the light-induced structuring process. Adjusting the parameters and variables above will lead to an optimized protocol for carrying out the described photopolymerization reaction, yielding optimal 3D-processing via lithography-based additive manufacturing technologies.

Preferably, the resin further comprises a thermal initiator and/or a catalyst for thermal curing, which are suitable for initiating a heat-triggered post-curing step or could be used to implement a secondary curing mechanism to yield dual-cure or hybrid networks (e.g., interpenetrating networks).

Recently, a strong focus has been put on hybrid resin systems composed of one or more additional material concepts different from the photopolymer network formation used in the light-structuring step of lithography-based additive manufacturing technologies (L-AMTs). Such promising hybrid material concepts show great potential for the formation of tough photopolymer resins for additive manufacturing and could yield photopolymerized materials with simultaneously high toughness and heat resistance. Hybrid resin systems can be defined as resin materials that exhibit various curing steps triggered by different impulses (e.g., a light-curing step followed by a subsequent heat-curing step - US 2016/0160077 A1) and/or materials that exhibit multiple hardening mechanisms (e.g., combined radical and cationic curing mechanisms or various radical curing mechanisms). Such formed photopolymer networks are considered hybrid materials composed of a first component and a second or multiple further components and typically represent a full interpenetrating network (IPN), a semi-IPN, a pseudo-IPN, a dual network or a polymer blend. In order to maintain reactivity toward light, thus ensure processability in L-AMTs, formulations with an effective content of light-curable components need to be formulated to yield materials with sufficient green strength. Green strength is the mechanical strength of a respective material (e.g., measured by tensile or bending test) received after the light-curing additive manufacturing step.

Preferably, the resin further comprises a second polymerizable system, suitable as a dual-curing system. This means that besides the previously described photopolymerization system, a second alternative system can be additionally incorporated for a second thermal polymerization process. This second system preferably comprises a mix of monomers, oligomers or prepolymers which contain a pair of functional groups suitable for a thermal polymerization process, which include epoxy with amines or alcohols, oxetane with amine or alcohols, isocyanates with alcohols, amines or carboxylic acids, amines with carboxylic acid, esters, anhydrides, aldehydes, acid chloride, alcohols with acid chlorides or halides, click chemistry reactions such as alkyne-azide, Michael additions, Diels-Alder reactions, maleimide and citraconimide derivatives with allyl and/or vinyl derivatives, alkenes or alkynes with thiols, peroxides, and silicon polymerization reactions.

Preferably, the resin further comprises one or more components selected from the group comprised of polymerization initiators, polymerization inhibitors, solvents, fillers, antioxidants, pigments, dyes, surface modifiers, flame retardants. The polymerization inhibitors are preferably quinones such as hydroquinones and benzoquinones, phenothiazines, diethylhydroylamine, 4-tert-butylcathecol, butylated hydroxytoluene, pyrogallol, TEMPO, preferably in amounts of 0.001 wt% to 1 wt%, more preferably 0.005 wt% to 0.5 wt%, and even more preferably 0.01 wt% to 1 wt% % based on the total weight of the at least one curable oligomer or prepolymer resin component and the reactive diluent.

To synthesize the reactive diluents comprised in the inventive resin composition, a two-step synthetic approach is used, which can start with a ring-opening reaction between a cycloaliphatic epoxide and (meth)acrylic acid, using a quaternary ammonium salt as the catalyst. Preferred epoxide starting materials include: cyclopentene oxide, cyclohexene oxide, cycloheptene oxide, cyclooctene oxide, 7-oxabicyclo[4.1.0]heptan-2-one, 9-oxobicyclo[6.1.0]non-4-ene, vitamin K₃ 2,3-epoxide, 2-methyl-1,2-cyclohexene oxide, 3-methyl-1,2cyclohexene oxide, 4-methyl-1,2-cyclohexene oxide, 4-vinyl-1-cyclohexene 1,2-epoxide, methyl 7-oxabicyclo[4.1.0]heptane-3-carboxylate, α -pinene oxide, limonene 1,2-epoxide, 6-oxabicyclo[3.1.0]hexan-3-one, 5-methyl-6-oxabicyclo[3.1.0]hexan-2-one, 3,4-epoxytetrahydrofuran and 3,4-epoxytetrahydrothiophene-1,1-dioxide. These epoxide starting materials are represented below by their structural formulae.

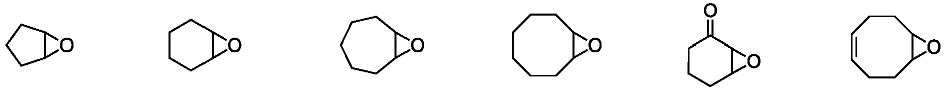

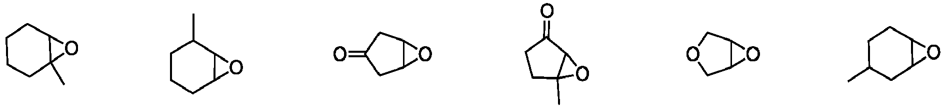

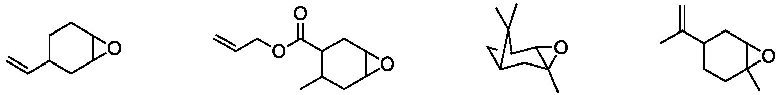

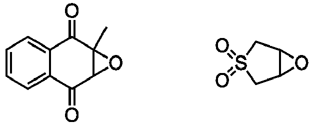

The obtained hydroxycycloaliphatic (meth)acrylates can then be reacted with an acyl chloride, carboxylic anhydride, chloroformate or a halogenated aliphatic compound. Preferred components for this reaction are: trimethylacetyl chloride, cyclohexanecarbonyl chloride, 2-ethylhexanoyl chloride, phenylacetyl chloride, hydrocinnamoyl chloride, cinnamoyl chloride, 4-fluorophenylacetal chloride, benzoyl chloride, phenoxyacetyl chloride, 4-methyl-1-cyclohexanecarboxylic acid, 1-methyl-1-cyclohexanecarboxylic acid, 4-chlorophenylacetyl chloride, propionyl chloride, p-tolyl-acetyl chloride, isobutyryl chloride, isovaleryl chloride, acetyl chloride, cyclobutanecarbonyl chloride, (4-tert-butylcyclohexyl)acetic acid, 4-tert-butylcyclohexanecarboxylic acid, cyclopropanecarbonyl chloride, 3,3-dimethylbutyryl chloride, 2-furoyl chloride, 3-furoyl chloride, 2-thiophenecarbonyl chloride, 5-methylfuran-2-carbonyl chloride, 3-nitrobenzoyl chloride, cycloheptanecarboxylic acid, hexanoic acid, octanoic acid, dodecanoic acid, pyrrole-3-carboxylic acid, 5-oxazolecarboxylic acid, 1H-pyrazole-3-carboxylic acid, 1-bromoadamantane, 1-adamantanecarboxylic acid, 1-adamantaneacetic acid, 3,5-dimethyladamantane-1-carboxylic acid, 9-anthracenecarboxylic acid, 2-naphthoic acid, biphenyl-4-carboxylic acid, 4'-methoxybiphenyl-4-carboxylic acid, isobutyl chloroformate, ethyl chloroformate, isopropyl chloroformate, menthyl chloroformate, phenyl chloroformate, allyl chloroformate, methyl chloroformate, cholesteryl chloroformate or cholesteryl hemisuccinate, camphanic chloride.

In a preferred alternative embodiment, in order to synthesize the reactive diluents comprised in the inventive resin composition, a different two-step synthetic approach starting with an amino alcohol is chosen, which after reacting with (meth)acryloyl chloride affords a (meth)acrylamide. Possible amino alcohols for this preferred reaction are: 2-aminocyclohexanol, 2-bromocyclopentanol, 2-aminocyclopentanol, 1-amino-2-indanol.

The obtained hydroxycycloaliphatic (meth)acrylamide can then be reacted with an acyl chloride, carboxylic acid, anhydride, chloroformate or a halogenated aliphatic compound. Possible components for this reaction are: trimethylacetyl chloride, cyclohexanecarbonyl chloride, 2-ethylhexanoyl chloride, phenylacetyl chloride, hydrocinnamoyl chloride, cinnamoyl chloride, 4-fluorophenylacetal chloride, benzoyl chloride, phenoxyacetyl chloride, 4-methyl-1-cyclohexanecarboxylic acid, 1-methyl-1-cyclohexanecarboxylic acid, 4-chlorophenylacetyl chloride, propionyl chloride, p-tolyl-acetyl chloride, isobutyryl chloride, isovaleryl chloride, acetyl chloride, cyclobutanecarbonyl chloride, (4-tert-butylcyclohexyl)acetic acid, 4-tert-butylcyclohexanecarboxylic acid, cyclopropanecarbonyl chloride, 3,3-dimethylbutyryl chloride, 2-furoyl chloride, 3-furoyl chloride, 2-thiophenecarbonyl chloride, 5-methylfuran-2-carbonyl chloride, 3-nitrobenzoyl chloride, cycloheptanecarboxylic acid, hexanoic acid, octanoic acid, dodecanoic acid, pyrrole-3-carboxylic acid, 5-oxazolecarboxylic acid, 1H-pyrazole-3-carboxylic acid, 1-bromoadamantane, 1-adamantanecarboxylic acid, 1-adamantaneacetic acid, 3,5-dimethyladamantane-1-carboxylic acid, 9-anthracenecarboxylic acid, 2-naphthoic acid, biphenyl-4-carboxylic acid, 4'-methoxybiphenyl-4-carboxylic acid, isobutyl chloroformate, ethyl chloroformate, isopropyl chloroformate, methyl chloroformate, phenyl chloroformate, allyl chloroformate, menthyl chloroformate, cholesteryl chloroformate or cholesteryl hemisuccinate, camphanic chloride.

The resulting reactive diluents can preferably be used in photopolymerization processes such as three-dimensional printing and stereolithography. More specifically, this class can be used as a replacement of 2-hydroxyethylmethacrylate (HEMA) presenting lower volatility, better (thermo)mechanical properties and higher reactivity.

Another aspect of the present invention is a method to polymerize the reactive diluents compound according to formula I with at least one oligomer, telechelic oligomer or prepolymer, to obtain a crosslinked polymer.

Preferably, the at least one curable oligomer or prepolymer resin component is a methacrylate resin such as: aliphatic urethane di(meth)acrylates (e.g., Ebecryl 8811, X-851-1066, Ebecryl 8809, Ebecryl 8408, Ebecryl 246, Miramer PU2100, Miramer SC2404, Miramer SC2565, Miramer PU2564, BR-571 MB, BR-541 MB, BR-7423GB, CN9001, CN965, CN981, CN8881 NS), ethoxylated bisphenol A di(meth)acrylates (e.g., Miramer 220, Miramer 2301), polyether urethane (meth)acrylates (e.g., Br-541 MB, BR582H15), hydrophobic urethane (meth)acrylates (e.g., BRC-443), polyester urethane (meth)acrylates (e.g., BR744 BT), polyester di(meth)acrylates (e.g., CN2608A, CN704, CN790), modified epoxy di(meth)acrylates (e.g., CN2003EU), oligomeric polycarbonate di(meth)acrylates.

The mentioned oligomer, telechelic oligomer or prepolymer to be used in the present invention can not only be difunctional, but also tri-, tetra-, penta-, hexafunctional or even having more functional sites or even having multiple functional sites on either end of the oligomer. Thus, it can also be not only linear, but also a type of branched polymer (e.g., star, dendrimer, comb, dendritic).

In a preferred embodiment, the cycloaliphatic compound according to formula I can also be mixed with other low-molecular weight monofunctional monomers, such as (meth)acrylates, (meth)acrylamides, vinylester and N-vinyl compounds. Some preferred monomers for mixing with the cycloaliphatic compound according to formula I are: isobornyl (meth)acrylate, cyclohexyl (meth)acrylate, trimethylcyclohexyl (meth)acrylate, glycerol formal (meth)acrylate, tricyclodecane methanol mono(meth)acrylate, 4-tert-butylcyclohexyl (meth)acrylate, benzyl (meth)acrylate, phenoxyethyl (meth)acrylate, cyclic trimethylolpropane formal (meth)acrylate, salicylate (meth)acrylates such as 2-(methacryloyloxy)benzoic acid cyclopentyl ester, 2-(methacryloyloxy)benzoic acid cyclohexyl ester, 2-(methacryloyloxy)benzoic acid-2-isopropyl-5-ethylcyclohexyl ester, 3-(methacryloyloxy)benzoic acid-2-isopropyl-5-methylcyclohexyl ester, 4-(methacryloyloxy)benzoic acid-2-isopropyl-5-methylcyclohexyl ester, 2-(methacryloyloxy)benzoic acid-3, 3, 5-trimethylcyclohexyl ester, 2-(acryloyloxy)benzoic acid-3, 3,5-trimethylcyclohexyl ester, 2-(methacryloyloxy)benzoic acid decahydronaphthalen-2-yl ester, 2- (methacryloyloxy)benzoic acid-1,3, 3-trimethyl-2-bicyclo[2.2.1 ]heptanyl ester, 2-(methacryloyloxy)benzoic acid-1 ,7, 7-trimethyl-2-bicyclo[2.2.1 ]heptanyl ester, 2- (methacryloyloxy)benzoic acid-bicyclo[2.2.1]heptan-2-yl methyl ester, 2-(methacryloyloxy)benzoic acid-2-cyclohexylethyl ester, 2-(methacryloyloxy)benzoic acid benzyl ester, 4-(methacryloyloxy)benzoic acid benzoate, 3-(methacryloyloxy)benzoic acid-4-isopropylbenzyl ester, 2-(acryloyloxy)benzoic acid benzyl ester, 2-(methacryloyloxy)benzoic acid phenethyl ester, 4-(methacryloyloxy)-3-methoxybenzoic acid-3-methoxybenzyl ester, 2-(methacryloyloxy)benzoic acid-1 -phenylethyl ester, 4-((methacryloyloxy)methyl)benzoic acid cycloheptyl ester and 2- (methacryloyloxy)benzoic acid cyclohexyl methyl ester, cholesteryl (meth)acrylate, biphenyl (meth)acrylate, phenyl acrylamide, diacetone acrylamide, t-butyl acrylamide, N-acryloyl morpholine, N-vinyl pyrrolidone, N-vinyl caprolactam, N-vinyl formamide, vinyl cinnamate, vinyl methyl oxazolidinone, and 2-(allyloxymethyl)acrylic acid methyl ester.

In another preferred embodiment of the present invention, the cycloaliphatic compound according to formula (I) can also be mixed with other difunctional and multifunctional (meth)acrylates and (meth)acrylamides with a low-molecular weight (below 500 g/mol), herein referred to as component C, to obtain crosslinked polymers. Components C are being added for better processability and higher green strength, if required. Multifunctional monomers making up components C are suitable for reasons of fast reaction rates and high crosslinking densities, improve heat resistance (e.g., higher T_{g}), but typically decrease toughness.

Some examples of these (meth)acrylates and (meth)acrylamides are: ethylene glycol di(meth)acrylate, 1,4-butanediol di(meth)acrylate, 1,3-butanediol di(meth)acrylate, triglycerol di(meth)acrylate, 1,6-hexanediol di(meth)acrylate, tetraethylene glycol di(meth)acrylate, di(ethylene glycol) di(meth)acrylate, tri(ethylene glycol) diacrylate, 1,10-decanediol di(meth)acrylate, neopentyl glycol diacrylate, tricyclo[5.2.1.0^{2,6}]decanedimethanol diacrylate, trimethylolpropane triacrylate, pentaerythritol tetraacrylate, piperazine diacrylamide, N,N'-methylenebis(acrylamide), N,N'-bis(acryloyl)cystamine, N,N'-(1,2-dihydroxyethylene)bisacrylamide and bisphenol A di(meth)acrylate.

The amount of component C preferably ranges from 5 wt% to 60 wt%, preferably from 5 wt% to 50 wt%, more preferably from 5 wt% to 40 wt% and even more preferably from 5 wt% to 35 wt% based on the total weight the at least one curable oligomer or prepolymer resin component and the reactive diluent in the composition.

The inventive method is preferably part of a high-temperature lithography-based process, wherein the inventive resin formulation comprises at least one photopolymerization initiator, preferably in amounts of 0.01 wt% to 10 wt%, more preferably of 0.1 wt% to 7 wt% and even more preferably of 2.5 wt% to 5 wt% based on the total weight of the at least one curable oligomer or prepolymer resin component and the reactive diluent in the composition. This photoinitiator should be suitable for radical polymerization upon irradiation, preferably within the wavelength spectrum of 150 nm to 1000 nm and more preferably between 200 nm to 550 nm. The photopolymerization method is particularly effective if the light exposure wavelength is adequate to activate the photoinitiator.

More preferably, this is part of an additive manufacturing process such as three-dimensional printing and the mixture is heated before being mixed with the photoinitiator. In some embodiments of the process, the resin composition is warmed up to the required process temperature, which is preferably 30 and 150°C, more preferably between 35°C and 100°C, and even more preferably between 40°C and 90°C, and afterwards it is exposed to light of an adequate wavelength.

Preferred photoinitiators are Norrish type I photoinitiators alone or in combination with each other and may be selected from the group consisting of alpha-hydroxyketones, phenylglyoxylates, benzyldimethylketals, alpha-aminoketones, mono- or bisacylphosphines, -phosphineoxides, mono-, bis- or tetraacylsilanes, -germanes, -stannanes, metallocenes. Some preferred examples are 2- hydroxy-2-methylpropiophenone, 1-hydroxycyclohexyl phenyl ketone, methyl phenyl glyoxylates, 2-benzyl-2-(dimethylamino)-4'-morpholinobutyrophenone, [1-(4-phenylsulfanylbenzoyl)heptylideneamino]benzoate, [1-[9-ethyl-6-(2-methylbenzoyl)carbazol-3- yljethylideneamino] acetate, 2,4,6-trimethylbenzoyldiphenylphosphine oxide (TPO), ethyl-(2,4,6- trimethylbenzoyl)phenyl phosphinate (TPO-L), phenylbis(2,4,6-trimethylbenzoyl)phosphine oxide (BAPO), ethyl (3-benzoyl-2,4,6-trimethylbenzoyl) (phenyl) phosphinate, bis(4- methoxybenzoyl)diethylgermane (BMDG), and bis (cyclopentadienyl) bis [2,6-dlfluoro-3-(1 -pyrryl) phenyl] titanium and/or polymeric type I photoinitiators such as Ominpol TP.

Other preferred photoinitiators are Norrish type II photoinitiators. Some preferred examples are benzophenones (e.g., benzophenone, 4-methyl benzophenone, 4,4'-bis(diethylamino)benzophenone), benzoin, diketones (e.g., 9,10-phenanthrenequinone, 1-phenyl- propane-1 ,2-dione, diacetyl or 4,4'-dichlorobenzil and/or derivatives thereof), and thioxanthones (e.g., chloropropoxythioxanthones, isopropylxanthones or 2,4-diethyl-9H-thioxanthen-9-one). Such type II photoinitiators may preferably be used in combination with coinitiators such as tertiary amines (e.g., aromatic tertiary amines such as N,N-dialkylaniline, -p-toluidine or - 3,5-xylidine, p-N,N- dialkylamino-phenylethanol, -benzoic acid derivatives, -benzaldehyde or triethanolamine.

A combination of Norrish type I and type II may also be used in some disclosures of this embodiment.

In other embodiments of the disclosed process, the photopolymerizable material might be subjected to a secondary post-curing process. This post curing process can be light-induced and, optionally, also heat-induced.

A preferred composition according to the present invention containing component A, component B, component C and a photoiniator may be chosen such that:

The amount of the photopolymerizable component A ranges from 5 wt% to 70 wt%, preferably from 5 wt% to 60 wt%, more preferably from 5% to 50% and even more preferably from 5% to 40% based on the total weight of components A and B in the formulation.

The amount of the photopolymerizable component B ranges from 30 wt% to 95 wt%, preferably from 40 wt% to 95 wt% and more preferably from 50 wt% to 95 wt% and even more preferably from 60 wt% to 95 wt% based on the total weight of components A and B in the formulation.

The amount of component C ranges from 5 wt% to 60 wt%, preferably from 5 wt% to 50 wt%, more preferably from 5 wt% to 40 wt% and even more preferably from 5 wt% to 35 wt% based on the total weight of components A and B in the formulation.

The amount of photoinitiator ranges from 0.01 wt% to 10 wt%, more preferably of 0.1 wt% to 7 wt% and even more preferably of 2.5 wt% to 5 wt% based on the total weight of components A and B in the formulation.

Another aspect of the present invention is directed to a method of manufacturing an object by 3D-printing, wherein a resin composition according to the first aspect of the invention is subjected to
a) a light-induced structuring step, optionally post-cured via a secondary light exposure, and
b) a subsequent heat-induced curing step.

In a preferred embodiment of the invention, single chemical compounds can alternatively be thermally homopolymerized in the heat-induced curing step leading to the formation of hybrid networks. Preferred chemical compounds for use in the heat-induced curing step include maleimide and citraconimide derivatives, epoxy derivatives and allyl derivatives. Preferably, a thermal initiator may be added for the heat-induced curing step, which include amines, azo compounds, alkali salts or peroxides, ranging from 0.01 wt% to 20 wt%, preferably from 0.1 wt% to 15 wt% and more preferably 0.2 wt% to 10 wt% based on the total weight of the at least one curable oligomer or prepolymer resin component and the reactive diluent.

Preferably, the referred second heat-induced curing step will only be activated once the formulation is subjected to heat, ideally at temperatures above 50°C, making the formulation stable at room temperature.

The resin composition can also be mixed with polymerization initiators, polymerization inhibitors, solvents, fillers, antioxidants, pigments, dyes, surface modifiers, flame retardants and mixtures thereof.

The polymerization inhibitors are preferably quinones such as hydroquinones and benzoquinones, phenothiazines, diethylhydroylamine, 4-tert-butylcathecol, butylated hydroxytoluene, pyrogallol, TEMPO, preferably in amounts of 0.001 wt% to 1 wt%, more preferably 0.005 wt% to 0.5 wt%, and even more preferably 0.01 wt% to 1 wt% % based on the total weight of the at least one curable oligomer or prepolymer resin component and the reactive diluent.

In photopolymerization-based printing processes, the photoresins are irradiated locally and with high resolution in a lithography-based process, with the irradiated areas curing to form a solid. The process takes place in stages or continuously and the previously prepared three-dimensional structures are built up. Here, the components are usually located on a construction platform. After completion of the three-dimensional printing process, the 3D components produced are contaminated with resin.

After a successful 3D printing process, the components can be finalized or refined. These processes are referred to as post-processing and include a variety of downstream processes to achieve the final component properties. One of the typical post-processing steps here is cleaning. This can be performed using centrifugal forces, with solvents as described in AT 525049 A4 using turbulent flows, directed jets, ultrasound, steam, aerosols and/or in pressure swing processes, using gases and/or directed gas flows (e.g., compressed air, heated compressed air).

After cleaning, one or more downstream processes are usually performed in order to arrive at the final 3D component. Typical downstream processes include drying, post-curing, coating with, for example, metals, semi-metals, ceramics and/or plastic coatings, mechanical post-processing of the 3D components, but also the removal of any support structures that may be present, assembly processes, the application of conductor tracks, the insertion of contact pins, coloring of components and much more. Drying steps can be carried out in air or other atmospheres, such as nitrogen or argon, at room temperature, at elevated temperatures, for example in ovens or belt ovens, by various compressed air technologies and/or by directed hot air.

Post-curing can be a thermal and/or radiation-induced process (e.g., UV light sources). The thermal processes described can take place at temperatures between 40 and 400°C. In the case of debinding and sintering or carbonization processes, the thermal processes can also take place at several hundred degrees and up to over 1000°C. The thermal exposure can take place in batches or continuously, for example in a belt furnace.

The same applies to radiation post-curing, which can also be carried out batchwise or continuously, for example in radiation belt ovens. All of those post-curing steps can be carried out in any order and any number of times in order to obtain the final properties of the 3D component.

The resin compositions of the present invention may be used for manufacturing various types of three-dimensional objects. Examples for applications of 3D printed objects include electronic, electric and electro-mechanic components, connectors, housings, automobile and aerospace sectors, electric mobility, communication technology, computer technology, military technology, medical devices, medical technology, consumer goods, sports industry, energy industry, printed electronics, dental and orthodontic applications.

### Examples

All chemicals were purchased from chemical suppliers (ABCR, Merck, TCI, Carbosynth) and used without further purification. The NMR spectra were recorded on a Bruker advanced Ultrashield 400 (¹H 400 MHz; ¹³C 101 MHz). Chemical shifts (δ) are given in parts per million (ppm) and were calibrated with internal standards of the deuterium labeled solvents (¹H 7.26 ppm; ¹³C 77.16 ppm). Proton multiplicities are denoted by the following abbreviations: s (singlet), d (duplet), t (triplet), q (quartet), p (quintet), m (multiplet), dd (doublet of duplets), dt (doublet of triplets), dq (doublet of quartets). Coupling constants (J) are presented in Hertz (Hz). TLC Thin layer chromatography) was performed using silica gel 60 aluminum plates containing fluorescent indicator from Carl Roth and detected either with UV light (254 nm) or staining in potassium permanganate (1.5 g KMnO₄, 6.6 g K₂CO₃, 1. 25 mL 10 % NaOH, 200 mL H₂O). Flash chromatography and vacuum dry chromatography were performed using silica gel technical grade (particle size 40-63 µm, 230-400 mesh) from Sulpeco.

Volatility can be assessed from the weight lost after 24 hours, and is preferably below 10%, more preferably below 5% and even more preferable below 1% at the process temperature (between 30 and 150°C). For the determination of the volatility of the compounds 25 mL flasks (Ø 30 mm) with approximately 2 grams of the test compound were placed in a Memmert oven at 50 °C and 80°C for 24 hours and the volatility was determined as the weight loss in percent (w/w).

Viscosity is another crucial parameter on the development of a reactive diluent. To obtain an effective reactive diluent, monomers providing a low viscosity need to be obtained. The referred viscosity is preferably between 5 and 100 mPa.s, more preferably between 5 and 50 mPa.s, and even more preferably 5 and 20 mPa.s. The presented viscosities refer to the process temperature which can be between 30-150°C. The viscosity was determined via rheology. Rheology measurements were performed using an Anton Paar MCR 102 rheometer, equipped with a P-PTD 200/GL Peltier glass plate and an H-PTD200 heating hood. For measuring the rheological properties, a temperature ramp experiment was performed from 25-100°C with a heating rate of 0.5°C min⁻¹, using a CP25 measurement system (cone plate with a 2° angle and 25 mm diameter) at a gap distance of 0.1 mm and with a shear rate of 60 s⁻¹.

Photo-DSC (differential scanning calorimetry) studies were performed using a Netzsch DSC 204 F1 containing an autosampler and coupled to an intracooler IC85 cooling device. The system was connected to an Omnicure LX500 controller with a 365 nm LED and the power intensity was set on the sample at 17 mW cm⁻² (measured using a Thorlabs SMC100 powermeter and the corresponding S310C thermal power head). The test samples were mixed in a vortex mixer for 3 minutes with 1% w/w of TPO-L (a commercially available photoinitiator) and then 1012 mg were transferred to an aluminum crucible without lid. All experiments were conducted under Nitrogen atmosphere (flow = 20 mL/min) at 50°C and the samples were irradiated for 5 minutes. Triplicate DSC measurements were performed for each example. The measured heat of polymerization (ΔH) was calculated by integrating the area under the curve from 0 to 200 s.

Here, tₘₐₓ is the time when maximum heat evolution was reached and t_{95%} corresponds to the point when 95% of the total heat of polymerization has evolved (0 s corresponds to the moment when light is triggered). The double bound conversion (DBC) was calculated from the experimentally obtained heat of polymerization and the theoretical polymerization enthalpy indicated in the literature (56 kJ mol⁻¹ for methacrylates).

For the (thermo)mechanical tests and water uptake tests, Example 1 or Reference 2 were mixed with two commercially available oligomers, namely Ebecryl 8811 (Allnex) a difunctional aliphatic urethane acrylate and BR-952 (Bomar) a difunctional aliphatic urethane methacrylate in different ratios.

For the sample preparation a speedmixer (DAC 150.1 FVZ-K, Hauschild) was used. 70 g of the total formulation was mixed at 3000 rpm for 10 minutes. After that the resin composition was degassed until no air bubbles were released.

Test specimens were printed on a Cubicure Caligma 200 DLP 405 nm. Light Pulse Duration was set to 6000, corresponding to 1.5 s total exposure time, and print speed to 20 mm/s. Processing temperature was 35°C, due to the high volatility of Reference 2. The settings used resulted in a curing depth of 220 pm.

Dynamic Mechanical Analysis (DMA) of the printed specimens measuring 50x5x1 mm³ was performed using a DMA8000 Dynamic Mechanical Analyzer from PerkinElmer. The measurements were performed using the tension mode, from 30-160 °C with a heating rate of 2°C min⁻¹ and frequency of 1 Hz. In these experiments the evolution of the storage modulus G' with increasing temperature was observed and the T_{g} was determined (maximum value for tan δ).

The water uptake was determined according to ISO 62. Each printed sample (10x10x1 mm³) was previously weighted and then placed in 25 mL of distilled water for 24 hours. Afterwards, the sample was cleaned and reweighted, and the water uptake was calculated in percentage from the weight gain (4 repetitions).

### Example 1: Synthesis of 2-hydroxycyclohexyl methacrylate (HCMA)

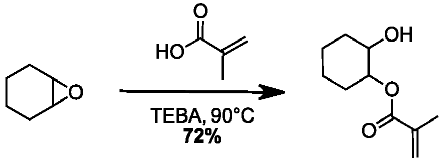

Methacrylic acid (308 g, 3.6 mol, 1 equiv.) and triethylbenzylammonium chloride (24.5 g, 0.11 mol, 0.03 equiv.) were mixed and placed under stirring. The mixture was slowly warmed to 70°C and once the target temperature was reached a mixture of hydroquinone (19.7 g, 0.18 mol, 0.05 equiv.) and cyclohexene oxide (352 g, 3.6 mol, 1 equiv.) was added dropwise over 3 hours. Upon completion of the addition the temperature was increased to 90°C and the reaction was stirred for 6 hours. Once the reaction was at room temperature, HCMA was distilled (2 mbar, boiling point (b.p.) = 115-121°C) and obtained in 72% yield as a colorless low-viscous oil (476 g, 2.6 mol) . ¹H NMR (CDCl₃) : δ = 1.23 - 1.43 (m, 4H), 1.68 - 1.75 (m, 2H), 1.94 - 1.95 (m, 3H), 2.01 - 2.08 (m, 2H), 3.58 - 3.65 (m, 1H), 4.60 - 4.68 (m, 1H), 5.56 - 5.58 (m, 1H), 6.10 - 6.13 (m, 1H) ppm. ¹³C NMR (CDCl₃): δ = 18.5, 23.8, 24.0, 30.0, 33.1, 72.9, 78.5, 125.8, 136.6, 167.7 ppm.

### Example 2: Synthesis of 2-(cyclohexylcarbonyloxy)cyclohexyl methacrylate

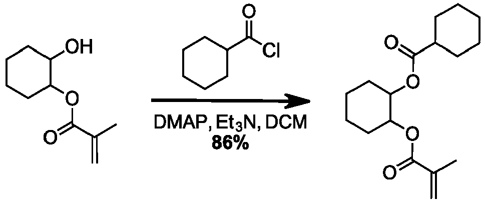

HCMA (13.4 g, 72.9 mmol, 1 equiv.) was dissolved in 35 mL of DCM and the triethylamine (8.1 g, 80.2 mmol, 1.1 equiv.) and DMAP (0.18 g, 1.5 mmol, 0.02 equiv.) were added. The reaction mixture was placed under stirring and under argon, cooled to 0°C and the cyclohexanecarbonyl chloride (11.4 g, 76.5 mmol, 1.05 equiv.) was added dropwise over the course of 20 minutes. After addition the reaction mixture was kept at 0°C for 10 minutes and then the reaction mixture was allowed to warm to room temperature. After 6 hours the reaction was filtered and the solid was washed with DCM, the filtrate was washed with 2 M HCl, saturated sodium hydrogen carbonate aqueous solution, brine and dried over sodium sulphate. After evaporation the crude material was purified via column chromatography.

(PE:EtOAc 15:1) and the desired product was obtained in 86% yield as a beige oil (18.5 g, 62.8 mmol) . ¹H NMR (CDCl₃) : δ = 1.17 - 1.30 (m, 3H), 1.30 - 1.49 (m, 6H), 1.56 - 1.63 (m, 1H), 1.65 - 1.75 (m, 4H), 1.75 - 1.85 (m, 2H), 1.88 - 1.92 (m, 3H), 1.95 - 2.01 (m, 1H), 2.04 - 2.11 (m, 1H), 2.14 - 2.29 (m, 1H), 4.73 - 5.00 (m, 2H), 5.44 - 5.64 (m, 1H), 5.97 - 6.14 (m, 1H) ppm. ¹³C NMR (CDCl₃) : δ = 18.4, 23.6, 23.6, 25.4, 25.6, 25.9, 28.9, 29.2, 30.2, 30.3, 43.4, 73.0, 74.2, 125.8, 136.4, 166.8, 175.6 ppm.

### Example 3: Synthesis of 2-(isopropenylcarbonyloxy)cyclohexyl 2,2-dimethylpropionate

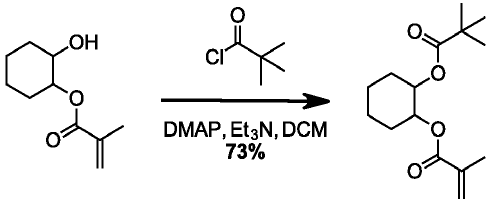

HCMA (3.0 g, 19.1 mmol, 1 equiv.) was dissolved in 20 mL of DCM and the triethylamine (3.1 g, 30 mmol, 1.7 equiv.) and DMAP (44 mg, 0.4 mmol, 0.02 equiv.) were added. The reaction mixture was placed under stirring and under argon, cooled to 0°C and the trimethylacetyl chloride (3.5 g, 28.0 mmol, 1.5 equiv.) was added dropwise over the course of 20 minutes. After addition the reaction mixture was kept at 0°C for 10 minutes and then the reaction mixture was allowed to warm to room temperature. After 8 hours the reaction was filtered and the solid was washed with DCM, the filtrate was washed with 2 M HCl, saturated sodium hydrogen carbonate aqueous solution, brine and dried over sodium sulphate. After evaporation the crude material was purified via column chromatography.

(PE:EtOAc 10:1) and the desired product was obtained in 73% yield as a beige oil (3.6 g, 13.4 mmol) . ¹H NMR (CDCl₃) : δ = 1.12 (s, 9H), 1.31 - 1.54 (m, 4H), 1.66 - 1.80 (m, 2H), 1.90 (s, 3H), 1.94 - 2.14 (m, 2H), 4.70 - 5.03 (m, 2H), 5.48 - 5.66 (m, 1H), 5.97 - 6.17 (m, 1H) ppm. ¹³C NMR (CDCl₃): δ = 18.4, 23.6, 23.6, 27.2, 30.2, 30.2, 38.8, 73.3, 74.1, 125.8, 136.4, 166.8, 178.0 ppm.

### Example 4: Synthesis of 2-(benzylcarbonyloxy)cyclohexyl methacrylate

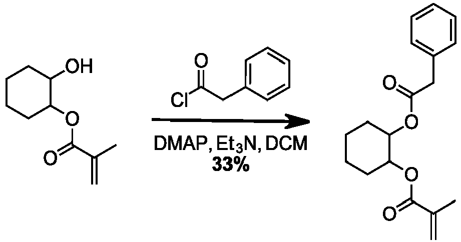

HCMA (6.2 g, 34.0 mmol, 1 equiv.) was dissolved in 20 mL of DCM and the triethylamine (8.3 g, 81.6 mmol, 2.4 equiv.), DMAP (0.2 g, 1.7 mmol, 0.05 equiv.) and 3000 ppm phenothiazine were added. The reaction mixture was placed under stirring and under argon, cooled to 0°C and the phenylacetyl chloride (10.7 g, 68.0 mmol, 2 equiv.) was added dropwise over 20 minutes. After addition the reaction mixture was kept at 0°C for 10 minutes and then the reaction mixture was allowed to warm to room temperature. After stirring overnight, the reaction was filtered and the solid was washed with DCM, the filtrate was washed with 2 M HCl, saturated sodium hydrogen carbonate aqueous solution, brine and dried over sodium sulphate. After evaporation the crude material was purified via column chromatography (Dichloromethane) and the desired product was obtained in 33% yield as a beige oil (3.4 g, 11.4 mmol). ¹H NMR (CDCl₃): δ = 1.22 - 1.36 (m, 2H), 1.61 - 1.67 (m, 2H), 1.72 - 1.75 (m, 3H), 1.88 - 2.05 (m, 2H), 3.48 (s, 2H), 5.36 - 5.39 (m, 1H), 5.87 - 5.91 (m, 1H), 7.12 - 7.22 (m, 5H) ppm. ¹³C NMR (CDCl₃): δ = 18.3, 23.5, 23.6, 30.2, 30.2, 41.8, 73.9, 74.2, 125.8, 127.1, 128.6, 129.2, 134.1, 136.2, 166.7, 171.0 ppm.

### Example 5: Synthesis of 2-(isopropenylcarbonyloxy)cyclohexyl 2-ethylhexanoate

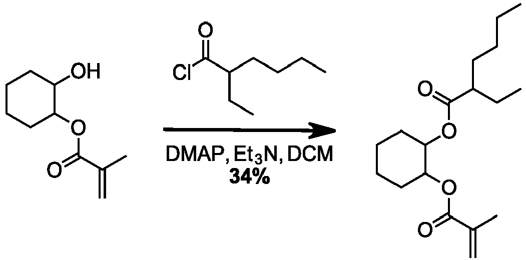

HCMA (4.4 g, 23.8 mmol, 1 equiv.) was dissolved in 6 mL of DCM and the triethylamine (4.1 g, 40.5 mmol, 1.7 equiv.) and DMAP (58 mg, 0.5 mmol, 0.02 equiv.) were added. The reaction mixture was placed under stirring and under argon, cooled to 0°C and the 2-ethylhexanoyl chloride (5.9 g, 35.7 mmol, 1.5 equiv.) was added dropwise over 20 minutes. After addition the reaction mixture was kept at 0°C for 10 minutes and then the reaction mixture was allowed to warm to room temperature. After 8 hours the reaction was filtered and the solid was washed with DCM, the filtrate was washed with 2 M HCl, saturated sodium hydrogen carbonate aqueous solution, brine and dried over sodium sulphate. After evaporation the crude material was purified via column chromatography.

(PE:EtOAc gradient, 25:1 to 10:1) and the desired product was obtained in 34% yield as a beige oil (2.5 g, 8.2 mmol). ¹H NMR (CDCl₃): δ = 0.81 - 0.87 (m, 6H), 1.13 - 1.60 (m, 12H), 1.67 - 1.78 (m, 2H), 1.90 (s, 3H), 1.93 - 2.12 (m, 2H), 2.12 - 2.27 (m, 1H), 4.75 - 4.97 (m, 2H), 5.46 - 5.56 (m, 1H), 6.00 - 6.13 (m, 1H) ppm. ¹³C NMR (CDCl₃): δ = 12.0, 14.0, 18.4, 22.7, 23.6, 25.6, 25.7, 29.6, 29.7, 30.3, 32.0, 47.8, 73.3, 74.0, 125.9, 136.4, 166.8, 175.9 ppm.

### Example 6: Determination of the volatility, photo-reactivity and viscosity

Volatility, viscosity and photo-reactivity are crucial parameters to assess the suitability of a compound as a reactive diluent. In the case of high-temperature processes, volatility is of special importance. Therefore, the previously referred parameters were determined for Examples 1-5 and for IBMA (Reference 1) and HEMA (Reference 2). The results are presented in Table 1.

Regarding viscosity it is observed that at room temperature examples 1-5 present higher viscosities than both references (8 mPa.s for Reference 1 and 6 mPa.s for Reference 2). Even though the viscosities at room temperature are higher, examples 1-3 present already a viscosity of approximately 50 mPa.s or lower, making them suitable reactive diluents at this temperature (53, 27 and 13 mPa.s, respectively). Examples 4 and 5 stand out because of their higher viscosity at room temperature (174 mPa.s and 110 mPa.s). However, when the temperature increases the viscosity decreases dramatically. At 40°C the viscosity of example 5 is 53 mPa.s making it already useful as a reactive diluent.

The volatility was determined at 50 and 80°C. It is clear from the results that the synthesized polymerizable monomers present a much lower volatility than reference 1 and reference 2. At 50°C examples 1-5 present a weight loss of 5% or lower, while reference 1 and 2 show a 12 % and 34 % weight loss, respectively. This difference is even more dramatic at 80°C, where reference 1 presents a weight loss 3 times higher than example 1, the most volatile monomer from examples 1-5 with a 20 % weight loss. Reference 2 presents the highest volatility with a weight loss more than 4 times higher than example 1.

From the photo-DSC experiments, the ΔH, tₘₐₓ, t_{95%} and DBC were determined to assess the photo-reactivity and for all examples except number 5 heat of polymerization was detected. Looking at the ΔH, examples 1-4 present similar values to Reference 1 (~40 kJ/mol), but a bit lower than Reference 2 (52 kJ/mol). The calculated DBC for examples 1-4 is between 80 and 85%, similar to Reference 1 (79%) but lower than Reference 2 (>99%). Examples 1 and 2 react faster than Reference 1 and Reference 2 since they reach tₘₐₓ and t_{95%} faster. Regarding example 3, a slower reaction is observed, while for example 4, the maximum heat of polymerization is reached faster (lower tₘₐₓ), but the reaction takes longer to reach completion (higher t_{95%}).

To summarize, after these first studies, all the newly synthesized reactive diluents are promising alternatives to reference 1 and 2. Examples 1 and 2 look the most promising due to the low viscosity, high reactivity and low volatility.

**Table 1**

| | Ref1 | Ref2 | Ex1 | Ex2 | Ex3 | Ex4 | Ex5 |
|---|---|---|---|---|---|---|---|
| Viscosity [mPa.s] at | | | | | | | |
| 25°C | 8 | 6 | 53 | 27 | 13 | 110 | 174 |
| 40°C | 6 | 4 | 26 | 17 | 9 | 53 | 101 |
| 50°C | 4 | 3 | 16 | 12 | 6 | 31 | 65 |
| 70°C | 3 | 2 | 7 | 7 | 4 | 13 | 34 |
| 90°C | 2 | 1 | 4 | 4 | 2 | 8 | 20 |
| 100°C | 2 | 1 | 3 | 4 | 2 | 7 | 19 |

| Volatility [weight loss %] at | | | | | | | |
|---|---|---|---|---|---|---|---|
| 50°C | 12 | 34 | 5 | 1 | 2.7 | 0.09 | 0.6 |
| 80°C | 65 | 89 | 20 | 14 | 21 | 17 | 1.9** |
| ΔH [kJ/mol] | 40 | 52 | 41 | 43 | 43 | 37 | * |
| tₘₐₓ [s] | 36 | 43 | 14 | 17 | 51 | 26 | * |
| t_{95%} [s] | 67 | 65 | 43 | 66 | 87 | 174 | * |
| DBC [%] | 79 | >99 | 80 | 85 | 85 | 72 | * |

| | | | | | | | |
|---|---|---|---|---|---|---|---|
| *No heat of polymerization was detected in the photo-DSC experiments **Monomer polymerized during the volatility test | | | | | | | |

### Example 7: (Thermo)mechanical tests and water uptake of crosslinked photopolymers

Looking at the high reactivity, low viscosity and low volatility of example 1, this compound was chosen for further testing as reactive diluent and compared with reference 2. The prepared specimens for this purpose are presented in Table 2.

**Table 2**

| % Component | | | | |
|---|---|---|---|---|
| | Ebecryl 8811 | X-851-1066 | Ref2 | Ex1 |
| Example 8 | 0 | 90 | 0 | 10 |
| Example 9 | 0 | 70 | 0 | 30 |
| Reference 3 | 0 | 90 | 10 | 0 |
| Reference 4 | 0 | 70 | 30 | 0 |
| Example 10 | 50 | 20 | 0 | 30 |
| Reference 5 | 50 | 20 | 30 | 0 |

For all specimens a DMA and the water uptake were determined, and the results are presented in Table 3.

The DMA tests show that the example 1-containing photopolymers present a higher T_{g} and G'_{30°C} than the respective reference 2-containing counterparts. For example, example 8 presents a T_{g} of 141°C and a G'_{30°C} of 3327 MPa, higher than the T_{g} of 136°C and G'_{30°C} of 2662 MPa observed for reference 3. It is also noticed that increasing the percentage of reference 2 has a more pronounced negative effect on the T_{g} than example 1, since when increasing the percentage of reference 2 from 10 to 30% (reference 3 vs reference 4) the T_{g} of the photopolymer decreases 13%, while the same variation in the content of example 1 (example 8 vs 9) leads to a decrease of only 4%. The water uptake tests clearly show that using example 1 as a reactive diluent leads to a much lower water uptake than reference 2. When the oligomer X-851-1066 is mixed with 10% reactive diluent (example 8 and reference 3), only a small difference of 0.1% in water uptake is observed. However, when the ratio of reactive diluent increases to 30%, the water uptake of the specimen containing example 1 (example 9) does not change, being 0.8%, while the water uptake of the reference 2 containing sample, reference 4, almost triplicates (increases from 0.9 to 2.6%). Example 10 and reference 5, photopolymers of Ebecryl 8811 and X-851-1066 with one of the reactive diluents, present a higher water uptake than the remaining, probably due to a higher water uptake of the resin Ebecryl 8811. However, the previously described trend is still observed since the water uptake of the reference 2-containing specimen is more than the double than the example 1-containing specimen (2.1 vs 5.6%).

**Table 3**

| | Ex8 | Ex9 | Ref3 | Ref4 | Ex10 | Ref5 |
|---|---|---|---|---|---|---|
| G'_{30°C} [MPa] | 3327 | 2836 | 2662 | 2911 | 1974 | 1684 |
| T_{g} [°C] | 141 | 135 | 136 | | 99 | 91 |
| Water uptake [%] | 0.8 | 0.8 | 0.9 | 2.6 | 2.1 | 5.6 |

To sum up, the displayed results clearly show that the disclosed compounds are much more suitable as reactive diluents at process temperatures in the range of 40-90 °C with viscosities in the range of 1-100 mPa.s and much lower volatility compared to state of the art reactive diluents (volatility is below 5% at 50 °C and below 20% at 80 °C for the tested diluents after 24 hours). Moreover, the tested reactive diluents show high reactivity leading to faster gelation compared to state of the art diluents (i.e., seen by faster tₘₐₓ in photo-DSC experiments, especially for Examples 1, 2 and 4). Fast gelation is an important characteristic for resin compositions for three-dimensional printing in order to keep processing times at a minimum and yield 3D-structures with high green strength.

The comparative study of example 1 with reference 2 showed that the example 1-containing photopolymers present a higher T_{g} and G'_{30°C} than the respective reference 2-containing counterparts. These improved properties can be explained by the rigid cycloaliphatic moieties represented by diluents according to formula I and the resulting cycloaliphatic nature and non-symmetric substitution pattern from compounds according to formula I additionally ensures low tendency for crystallization, thus rendering these inventive compounds particularly useful as reactive diluents for resin compositions in three-dimensional printing at elevated temperatures (e.g., 30-150 °C). Improved (thermo)mechanical performance is beneficial for the 3D-printing process itself, but also especially important for the final application of the 3D-printed structures.

Furthermore, the water uptake tests clearly show that using example 1 as a reactive diluent leads to a much lower water uptake in the final polymer than by using the compound of reference 2. This can be explained by the larger cycloaliphatic moiety giving the reactive diluent of example 1 a more hydrophobic nature, thus this holds also true for other examples according to formula I. These described benefits make the disclosed class of reactive diluents particularly promising as reactive diluents in resin compositions for three-dimensional printing.

## Claims

1. Resin composition for three-dimensional printing, the resin composition comprising
a) at least one curable oligomer or prepolymer resin component (compound B), and
b) a reactive diluent (compound A), the reactive diluent having one or more chemical species being a cycloaliphatic compound according to formula (I): 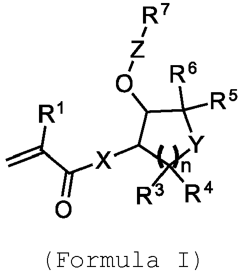 wherein:
n is an integer between 1 and 5;
R¹ is a hydrogen or a methyl group;
X is an -O- or -NR₂-;
R² is an H or an alkyl group;
R³, R⁴, R⁵ and R⁶ are independently H, a linear or branched C1-C20 aliphatic group or a C5-C8 cycloaliphatic group; or R³ and R⁴ together and/or R⁵ and R⁶ together represent a carbonyl group;
Y is carbon, oxygen, sulfur, sulfone, or absent;
Z is a carbonyl group, or forms a carbonate (-CO-O-) or
carbamate group (-CO-NR⁸), or is absent;
R⁷, R⁸ are independently H, a linear or branched C1-C20 aliphatic group, C5-C8 cycloaliphatic group, a heterocycle such as a tetrahydrothiophene, or a substituted or unsubstituted aromatic group, wherein the substituents of the aromatic group can be H, a linear or branched C1-C10 aliphatic group, methoxy or ethoxy groups, or halogens such as F, Cl, Br or I.

2. Resin composition according to claim 1, **characterized in that** in formula (I) n is 1 or 2.

3. Resin composition according to claim 1, **characterized in that** in formula (I) n is 2 or 3 when Y is absent.

4. Resin composition according to claim 1, 2 or 3, **characterized in that** the at least one curable oligomer or prepolymer resin component is chosen from (meth)acrylate, (meth)acrylamide, vinyl ester, vinyl ether, vinyl amide, vinyl carbonate, styrene, itaconate, fumarate, maleimide and derivatives thereof.

5. Resin composition according to any one of claims 1 to 4, **characterized in that** the at least one curable oligomer or prepolymer resin component comprises one or more mono- or multifunctional chemical species.

6. Resin composition according to any one of claims 1 to 5, **characterized in that** the molecular weight of the at least one curable oligomer or prepolymer resin component is higher than 450 g/mol and preferably higher than 1000 g/mol, as determined by either GPC or SEC.

7. Resin composition according to any one of claims 1 to 6, **characterized in that** the amount of reactive diluent ranges from 5 wt% to 80 wt%, preferably from 5 wt% to 60 wt%, more preferably from 5% to 50% and even more preferably from 5 wt% to 40 wt%, based on the total weight of the at least one curable oligomer or prepolymer resin component and the reactive diluent.

8. Resin composition according to any one of claims 1 to 7, **characterized in that** the amount of the at least one curable oligomer or prepolymer resin component ranges from 20 wt% to 95 wt%, preferably from 40 wt% to 95 wt% and more preferably from 50 wt% to 95 wt% and even more preferably from 60 wt% to 95 wt%, based on the total weight of the at least one curable oligomer or prepolymer resin component and the reactive diluent.

9. Resin composition according to any one of claims 1 to 8, characterized that the reactive diluent has a weight loss at a temperature of up to 50°C over 24 hours of lower than 10 %, preferably lower than 5 %, and more preferably lower than 1 %.

10. Resin composition according to any one of claims 1 to 9, characterized that the reactive diluent has a viscosity of 1 to 60 mPa.s, preferably of 1 to 40 mPa.s, and more preferably of 1 to 20 mPa.s, at a processing temperature of between 30 and 150°C.

11. Resin composition according to any one of claims 1 to 10, characterized that in formula (I) R⁷ is selected from the following structures, where the dotted lines represent the position of the bond to Z in formula (I):

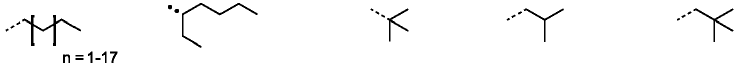

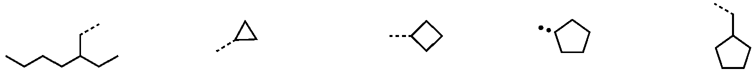

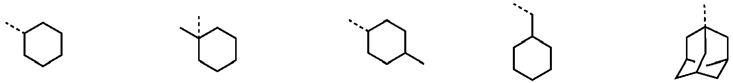

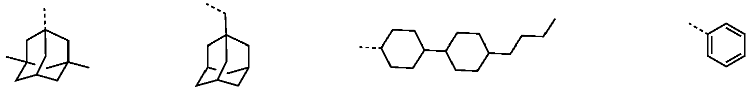

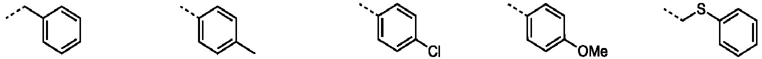

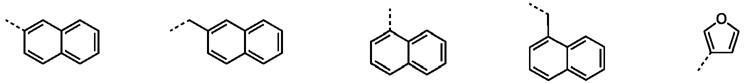

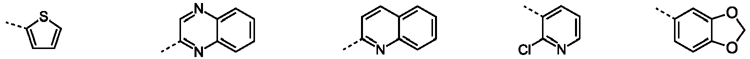

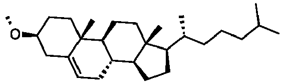

12. Resin composition according to any one of claims 1 to 11, **characterized in that** the reactive diluent according to the general formula (I) is selected from the group comprised of the following compounds:
2-hydroxycyclohexyl methacrylate (Ex 1) 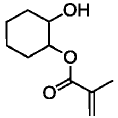 **Ex 1**
2-(cyclohexylcarbonyloxy)cyclohexyl methacrylate (Ex 2) 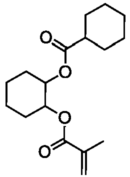 **Ex 2**
2-(isopropenylcarbonyloxy)cyclohexyl 2,2-dimethylpropionate (Ex 3) 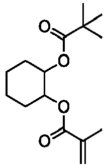 **Ex 3**
2-(benzylcarbonyloxy)cyclohexyl methacrylate (Ex 4) 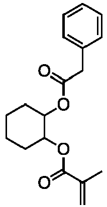 **Ex 4**
2-(isopropenylcarbonyloxy)cyclohexyl 2-ethylhexanoate (Ex 5) 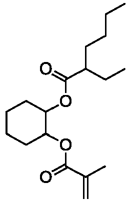 **Ex 5**

13. Resin composition according to any one of claims 1 to 12, **characterized in that** it further comprises at least one polymerization photoinitiator, preferably in amounts of 0.01 wt% to 10 wt%, preferably 0.1 wt% to 7 wt%, more preferably 0.2 wt% to 5 wt%, based on the total weight of the at least one curable oligomer or prepolymer resin component and the reactive diluent in the composition.

14. Resin composition according to any one of claims 1 to 13, **characterized in that** it further comprises a polymerization thermal initiator and/or a catalyst for thermal curing.

15. Resin composition according to any one of claims 1 to 14, **characterized in that** it further comprises a second polymerizable system, suitable as a dual-curing system.

16. Resin composition according to any one of claims 1 to 15, **characterized in that** it further comprises one or more components selected from the group comprised of polymerization initiators, polymerization inhibitors, solvents, fillers, antioxidants, pigments, dyes, surface modifiers, and flame retardants.

17. A method of manufacturing an object by 3D-printing, wherein a resin composition according to any one of claims 1 to 16 is subjected to
a) a light-induced structuring step, optionally precured via a secondary light exposure, and
b) a subsequent heat-induced curing step.

18. Method according to claim 17, **characterized in that** the light-induced structuring step is carried out at a processing temperature of the resin composition of between 30°C and 150°C, more preferably between 35°C and 100°C, even more preferably between 40°C and 90°C.
